# EUROPEAN PATENT SPECIFICATION

(11) **EP 3 292 835 B1**
(45) Date of publication and mention of the grant of the patent: **23.06.2021**
(21) Application number: 17190097.0
(22) Date of filing: 08.09.2017
(51) Int. Cl.: A61B 34/20

(54) **ENT IMAGE REGISTRATION**
HNO-BILDREGISTRIERUNG
ENREGISTREMENT D'IMAGE OTO-RHINOLARYNGOLOGIQUE

(30) Priority: 08.09.2016 US 201662384823 P; 10.08.2017 US 201715674380
(43) Date of publication of application: 14.03.2018
(73) Proprietor: Biosense Webster (Israel) Ltd., 2066717 Yokneam (IL)
(72) Inventor: BUSTAN, Itamar, 2066717 Yokneam (IL); PINSKY, Yoav, 2066717 Yokneam (IL); ZOABI, Akram, 2066717 Yokneam (IL); GOVARI, Assaf, 2066717 Yokneam (IL); SHILEMAY, Moshe I., 2650120 Haifa (IL)
(74) Representative: Carpmaels & Ransford LLP

(56) References cited:
- WO-A1-2016/007595
- CN-A- 103 251 457
- US-A- 6 096 050
- US-A1- 2016 073 933

## Description

### FIELD OF THE INVENTION

This invention relates generally to registration of images, and specifically to images generated with different modalities that may be used for image-guided surgery.

### BACKGROUND OF THE INVENTION

In image-guided surgery (IGS) a medical practitioner uses instruments that are tracked in real time so that positions and/or orientations of the instruments may be presented on images of a patient's anatomy during a surgical procedure. In some cases both the tracking and the imaging of the patient's anatomy may be implemented by one modality, such as fluoroscopy. However, because fluoroscopy uses ionizing radiation, its use should be minimized. Consequently in many scenarios an image of the patient is prepared in one modality, such as magnetic resonance imaging (MRI) or computerized tomography (CT) fluoroscopy, and the instrument tracking uses a different modality, such as electromagnetic tracking.

In order for the tracking to be effective, frames of reference of the two modalities have to be registered with each other. In the case of ear, nose, and throat (ENT) surgery, especially in the region of the sinuses, accurate registration is critical because of the proximity of the sinuses to the brain and other organs such as the optic nerves.

US 6096050 describes a system and process for correlating points on a spatial body to corresponding points on a database body formed from pre-aquired images of the spatial body. The system and process involve matching the spatial points with the corresponding database points using relative positional information between the spatial points and the database points. A preferred method, in the application, for selecting spatial points involves placing small markers of a contrast material, such as a capsule filled with a copper sulphate solution for MRI on anatomical features of the patient, such as the forehead or back of the head. These markers can comprise the selected spatial points on a patient and the same points will appear on images and can be easily identified to provide corresponding database points.

### SUMMARY OF THE INVENTION

The invention is set out in the appended set of claims.

### BRIEF DESCRIPTION OF THE DRAWINGS

Fig. 1 is a schematic diagram illustrating an ENT (ear, nose, and throat) registration system, according to an embodiment of the present invention;
Fig. 2 is a flowchart of steps of a process for the system of Fig. 1, according to an embodiment of the present invention;
Figs. 3 - 5 are schematic diagrams illustrating the steps of the flowchart of Fig. 2;
Fig. 6 is a schematic diagram illustrating an alternative ENT registration system, according to an embodiment of the present invention;
Fig. 7 is a flowchart of steps of an alternative process for the system of Fig. 6, according to an embodiment of the present invention;
Fig. 8 is a schematic diagram illustrating a step of the flowchart of Fig. 7; and
Fig. 9 is a flowchart showing steps for finding centers of the eyes of a patient in a CT image of the patient.

### DETAILED DESCRIPTION OF EMBODIMENTS

### Overview

In a typical registration procedure between a CT image and a magnetic tracking system the locations of a number of different points, accessible to both systems, are acquired in both systems. As one example, the nose tip may be identified in the CT image, and the location of the nose tip may also be acquired in the magnetic tracking system. Once pairs of such points have been acquired, a theorem such as the iterative closest point (ICP) theorem, or a variation of the ICP theorem, uses a cost function to estimate the transformation, of rotation and/or translation, that best aligns, i.e., registers, the two sets of points. Typically, the process is iterated, for example by increasing the numbers of pairs of points, to improve the accuracy of the transformation.

Because of the characteristics of the CT image, the locations of external features of a patient undergoing an ENT procedure, i.e., the locations of different skin regions such as the nose tip or the earlobes, are well defined. However, in a magnetic tracking system, where a magnetic sensor is positioned on the region, the location may be poorly defined. For example, an earlobe may move when the magnetic sensor is positioned on it. For accurate registration between the two systems, it is important that regions, accessible to the magnetic system and having a low probability of movement when their location is acquired in the system, are used. Embodiments of the present invention provide methods that assist an operator to identify and use such regions.

In one embodiment the CT image of a subject is analyzed to identify respective locations of a left eye and a right eye of the subject in the image, so as to define a first line segment joining the respective locations. A voxel subset of the image is identified, the subset comprising voxels that overlay bony sections of the subject's head, and that lie on a second line segment parallel to the first line segment and on a third line segment orthogonal to the first line segment.

A magnetic tracking system that is configured to measure positions on the subject's head is activated. A probe, operative in the magnetic tracking system, is positioned in proximity to the bony sections so as to measure magnetic-system-positions of a surface of the head overlaying the bony sections.

A processor forms a correspondence between the magnetic-system-positions and the voxel subset and generates a registration between the CT image and the magnetic tracking system in response to the correspondence.

In an alternative embodiment the CT image of the subject is analyzed to identify respective locations of a left eye and a right eye of the subject in the image, so as to define a first line segment joining the respective locations and a second line segment orthogonally cutting the first line segment, the two line segments dividing the image into regions.

A magnetic tracking system that is configured to measure positions on the subject's head is activated. A probe, operative in the magnetic tracking system, is positioned in proximity to a surface of the head corresponding to the regions, so as to measure magnetic-system-positions of the surface.

A processor counts a number of the magnetic-system-positions measured, and when the count of the magnetic-system-positions of a given region exceeds a preset threshold for the given region, provides an indication thereof and forms a correspondence between the magnetic-system-positions and the voxels of the image. The processor generates a registration between the CT image and the magnetic tracking system in response to the correspondence.

### Detailed Description

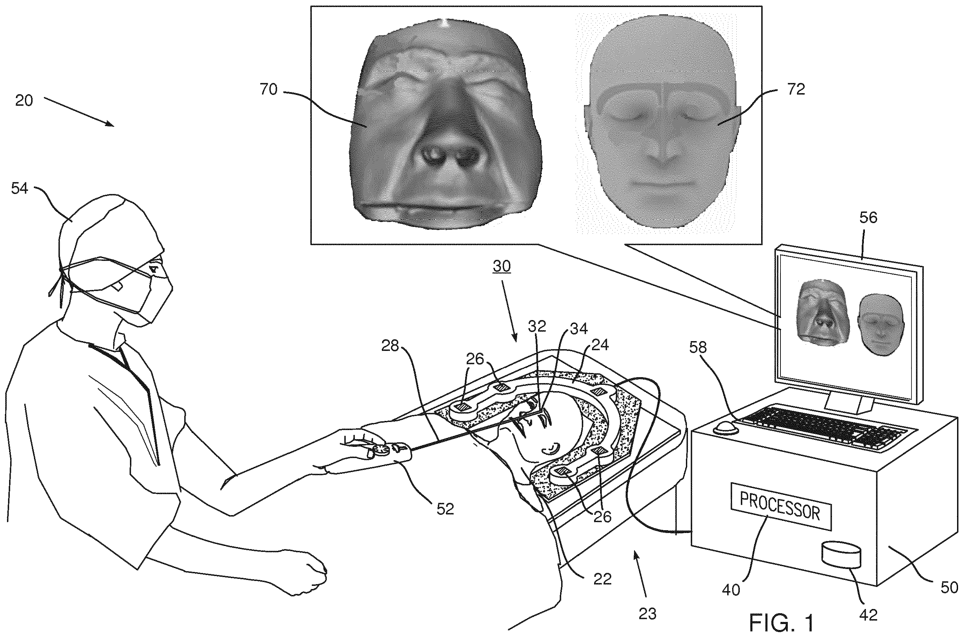

Reference is now made to Fig. 1, which is a schematic illustration of an ENT (ear, nose, and throat) image registration system 20, according to an embodiment of the present invention. In the following description registration system 20 is assumed to be used prior to performance of a nasal sinus procedure on a patient 22. System 20 registers frames of reference of a CT (computerized tomography) image of patient 22, herein assumed by way of example to comprise a fluoroscopic CT image, and of a magnetic tracking system 23 used to track a magnetic sensor in proximity to the patient.

In system 20, and during the subsequent sinus procedure, a magnetic radiator assembly 24, comprised in the magnetic tracking system, is positioned beneath the patient's head. Assembly 24 comprises magnetic field radiators 26 which are fixed in position and which transmit alternating sinusoidal magnetic fields into a region 30 wherein the head of patient 22 is located. By way of example, radiators 26 of assembly 24 are arranged in an approximately horseshoe shape around the head of patient 22. However, alternate configurations for the radiators of assembly 24 will be apparent to those having ordinary skill in the art.

Prior to the procedure, for the registration performed by system 20, a distal end 34 of a probe 28, having a magnetic sensor 32 at the distal end, is touched at different regions of the skin of patient 22. The signals induced in the sensor in response to its interaction with the magnetic fields enable the position of distal end 34 to be tracked, once assembly 24 has been calibrated. A probe controller 52, held by a physician 54 operating system 20, is connected to the proximal end of probe 28, the controller allowing the physician to control acquisition of the signals from sensor 32. The Carto® system produced by Biosense Webster, of Diamond Bar, CA, uses a system similar to that described herein for finding the location and orientation of a coil in a region irradiated by magnetic fields.

Elements of system 20, including radiators 26, are controlled by a system processor 40, comprising a processing unit communicating with one or more memories. Processor 40 may be mounted in a console 50, which comprises operating controls 58 that typically include a keypad and/or a pointing device such as a mouse or trackball. Console 50 connects to the radiators via a cable and/or wirelessly, and also connects to other elements of system 20, such as controller 52 of probe 28. Physician 54 uses controller 52 and operating controls 58 to interact with the processor while performing the registration of system 20. During the registration process, a surface image 70 and a face sketch 72, for viewing by the physician, are presented on a screen 56. The functions of surface image 70 and face sketch 72 are described in detail below. (Subsequent to the registration process, physician 54 uses the operating controls to interact with the processor while performing the procedure, and the processor may present results of the procedure on screen 56.)

Processor 40 uses software stored in a memory 42 to operate system 20. The software may be downloaded to processor 40 in electronic form, over a network, for example, or it may, alternatively or additionally, be provided and/or stored on non-transitory tangible media, such as magnetic, optical, or electronic memory.

Processor 40 uses the software, *inter alia,* to operate magnetic radiators 26 of assembly 24. As stated above the radiators transmit sinusoidal alternating magnetic fields of different frequencies into region 30, including the head of patient 22, and the fields from the radiators induce signals in sensor 32. The processor analyzes the signals to derive location and orientation values, measured with respect to a frame of reference defined by the assembly, for the sensor and thus for the distal end of probe 28.

Prior to performance of the procedure a CT image of patient 22 is acquired for use by system 20. Data of the CT image is stored in memory 42 for subsequent retrieval by processor 40. As is described below, the processor uses the stored data to present surface image 70 on screen 56.

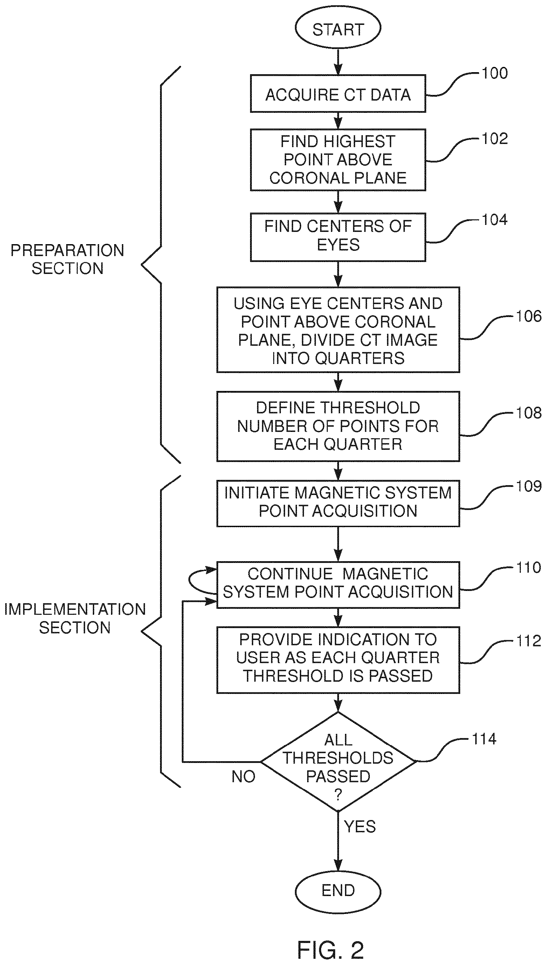

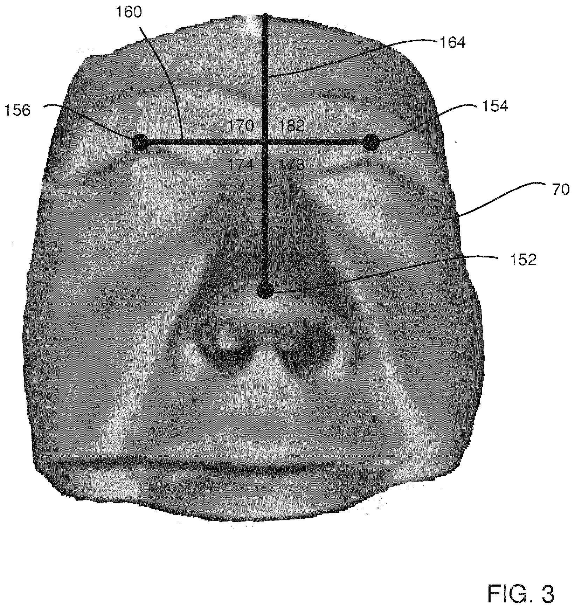

Fig. 2 is a flowchart of steps of a process implemented in system 20, and Fig. 3 is a schematic figure illustrating first steps of the flowchart, according to an embodiment of the present invention. The flowchart is divided into two sections: a first preparation section comprising steps 100 - 108, and a subsequent implementation section comprising steps 109 - 114. The implementation section of the flowchart may be performed days, or even weeks, after completion of the preparation section.

In an initial step 100 a CT image of the head of patient 22 is generated, and the acquired data of the image is stored in memory 42.

In a first image analysis step 102 processor 40 accesses the stored CT data and generates surface image 70 of the head of the patient. The processor then finds a highest point 152 of the surface image that is above the coronal plane of the patient. In accordance with the invention, the highest point corresponds to the nose tip of the patient.

In a second image analysis step 104 the processor 40 finds centers 154, 156 of the eyes of the patient. An article "Locating the eyes in CT brain scan data" in the Proceedings of the 6th international conference on Industrial and Engineering Applications of Artificial Intelligence and Expert Systems, pgs. 507-517, published in 1993, describes one method for finding the centers of the eyes, and the article is incorporated herein by reference. Other methods for finding the centers will be apparent to those having ordinary skill in the art, and all such methods are assumed to be comprised within the scope of the present invention. A description of a method for finding centers of the eyes of a patient, in an image of the patient, is provided in the Appendix below.

In a construction step 106 the processor constructs a line 160 joining the two eye centers, found in step 104. In addition, the processor constructs a line 164 orthogonal to line 160, parallel to the coronal plane, and passing through a projection of the highest point found in step 102. As illustrated in Fig. 3, the two lines divide the corresponding surface image into four quarter regions 170, 174, 178, 182, also herein termed quadrants.

In a threshold definition step 108, processor 40 stores minimum numbers of points to be acquired in each of quadrants 170, 174, 178, 182. In one embodiment the minimum numbers are respectively 20, 12, 12, and 20. However, the minimum numbers may be smaller or larger than these values, and may be the same or different for all sections. The inventors have found the values for the number of points stated above give satisfactory results, and those with ordinary skill in the art will be able to determine other sets of values, that give satisfactory results, without undue experimentation.

Step 108 completes the preparation section of the flowchart.

In an initial magnetic system point acquisition step 109, which is the beginning of the implementation section of the flowchart, physician 54 activates probe 28 so that signals from magnetic sensor 32 (at the probe's distal end) may be acquired by processor 40. Typically, signals are acquired when the physician has positioned distal end 34 of the probe on a desired portion of the patient's skin, and at the same time activates system 20, for example using a control in controller 52 of the probe, to save the acquired signals to memory 42.

In step 109 the physician positions the distal end at a preset number of "landmark" points on the patient's skin. The landmark points correspond to predetermined CT positions in the CT image that processor 40 is able to identify. In one embodiment four landmark points, comprising a point below the tip of the patient's nose, the left and right sides of the patient's face besides the eyes, and a point between the eyes are used, and signals from magnetic sensor 32 are acquired at these points.

Once the magnetic sensor signals have been acquired, the processor calculates respective magnetic positions in the magnetic assembly frame of reference, so generating four ordered pairs of positions, each ordered pair being a correspondence and having the form (magnetic position, CT position).

Processor 40 uses the set of ordered pairs to generate a preliminary registration, i.e., a transformation comprising a translation and/or a rotation, that aligns the CT system with the magnetic assembly.

While the embodiment described above uses four landmark points, the inventors believe that fewer than four points, possible only two points, may be sufficient to generate the preliminary registration.

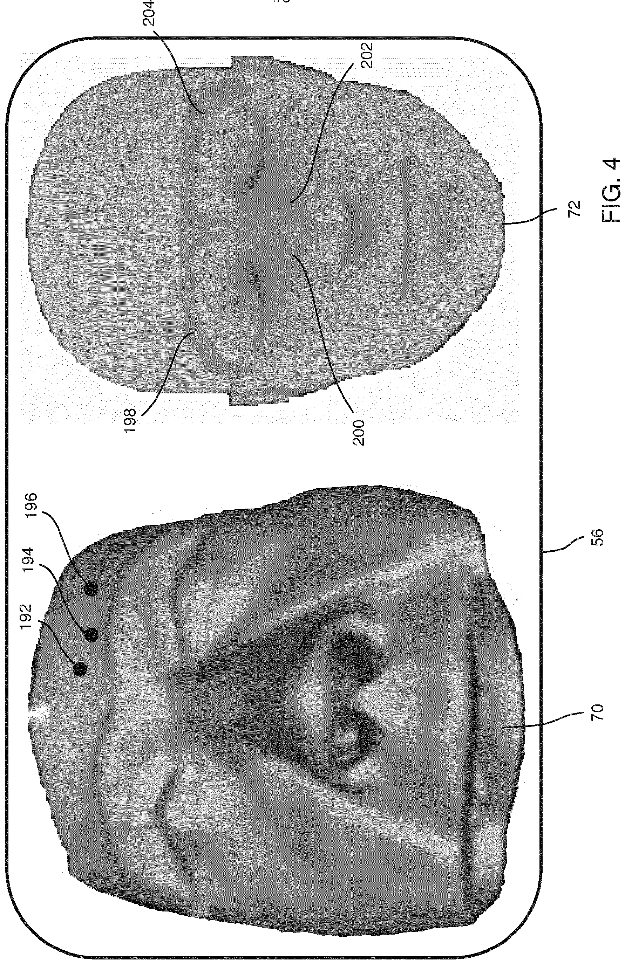

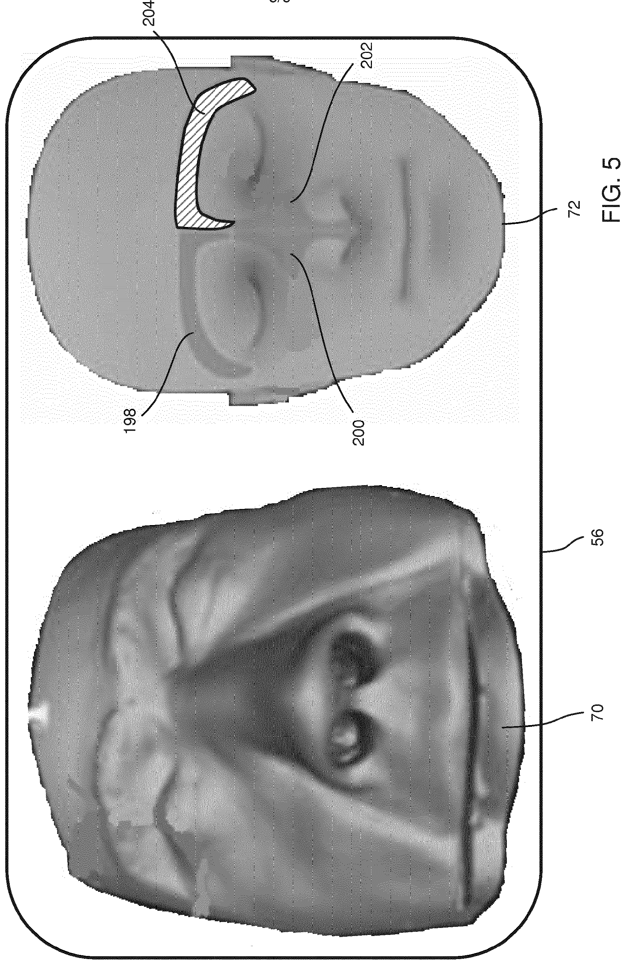

Fig. 4 illustrates an initial display of surface image 70 and face sketch 72 on screen 56, and Fig. 5 illustrates a subsequent display of the surface image and of the sketch, according to an embodiment of the present invention. As is described below, the sketch is used as a visual indicator for a user of system 20, to indicate when sufficient data has been collected for accurate registration of the frames of reference of the CT image and of the magnetic tracking system (as compared with the preliminary registration found in step 109).

Returning to the flowchart, in a continuing acquisition step 110 the processor displays the surface image and the human face sketch on screen 56, as is illustrated in Fig. 4. Physician 54 continues to acquire signals from magnetic sensor 32, and processor 40 saves the acquired signals to memory 42. In some embodiments a notice is displayed on screen 56, suggesting that the physician acquires signals when distal end 34 is positioned close to bony features of the patient, such as the bridge of the nose and the eyebrows or forehead.

Each time signals are acquired, a respective mark is placed on surface image 150, in a region corresponding approximately to the location of distal end 34 as determined by the preliminary registration of step 109. Fig. 4 illustrates, by way of example, three marks 192, 194, and 196.

Each time signals are acquired the processor uses the location determined by the signals to update the ICP theorem, using the new location as an addition to a source cloud of points. (Surface image 70 corresponds to a reference cloud of points used by the ICP theorem.)

In addition, each time signals are acquired the processor increments a counter for the quarter region where the signal is acquired.

In a continuing step 112, the processor checks the counters for each quarter region, and as a threshold of acquired locations is reached for the region, the processor provides an indication to the physician that the region threshold has been reached.

In an embodiment of the present invention the indication provided is a visual indication wherein a portion of sketch 70, that was initially gray, is altered visually, such as by being colored or shaded. Figs. 4 and 5 illustrate four gray areas 198, 200, 202, and 204, respectively corresponding to quarter regions 170, 174, 178, 182, that are used as indication areas. Fig. 5 illustrates that area 204 has been shaded, indicating that the threshold for quarter region 182 has been reached.

In a condition step 114 the physician checks, by inspection of the sketch, if the thresholds of all quarter regions have been reached. If the check returns positive, the physician may stop acquiring locations with probe 28. If the check returns negative, i.e., if one or more visual indications of reaching a threshold are not provided, the flowchart returns to step 110 and the physician continues acquiring points with probe 28.

The inventors have found that implementation of system 20, as described above, provides a fast, efficient, and accurate registration of the magnetic system frame of reference with the CT imaging frame of reference.

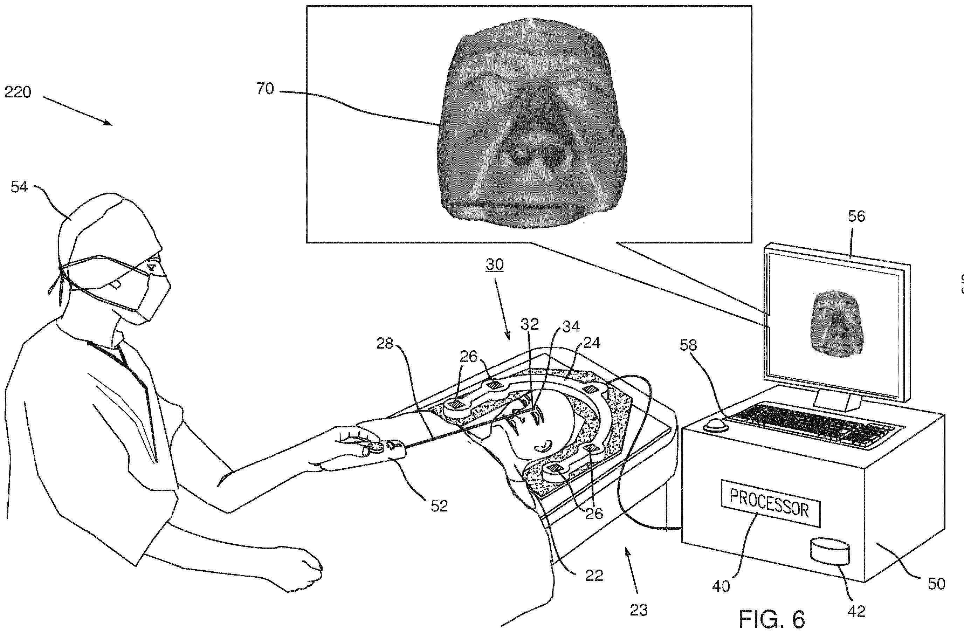

Reference is now made to Fig. 6, which is a schematic illustration of an alternative ENT image registration system 220, according to an embodiment of the present invention. Apart from the differences described below, the operation of system 220 is generally similar to that of system 20 (Figs. 1 - 5), and elements indicated by the same reference numerals in both systems 20 and 220 are generally similar in construction and in operation.

In contrast to system 20, in system 220 no visual indicator such as sketch 72 is presented on screen 56, so that the display on the screen may be only surface image 70. Also in contrast to the steps of the flowchart of Fig. 2 used by system 20 to register the two frames of reference, system 220 uses steps of a different flowchart, as described hereinbelow.

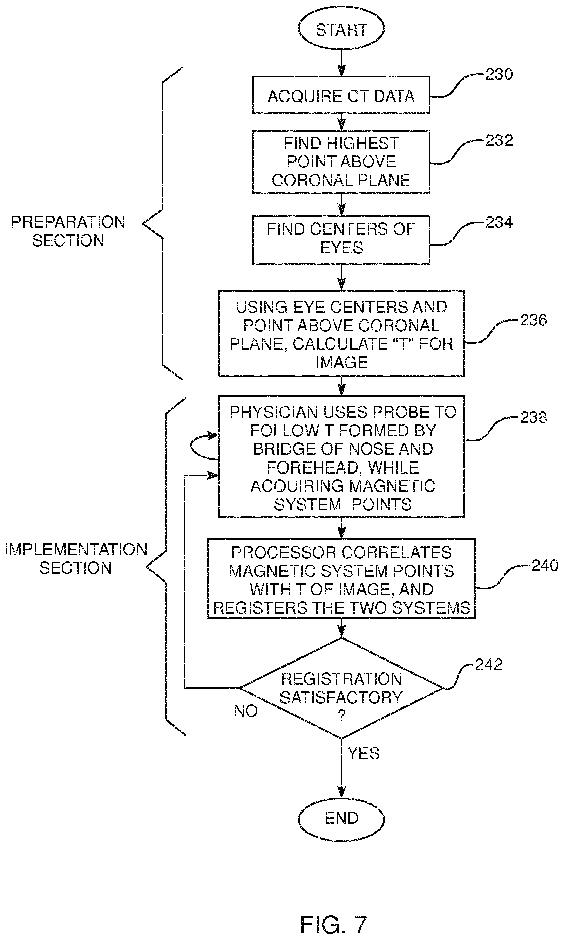

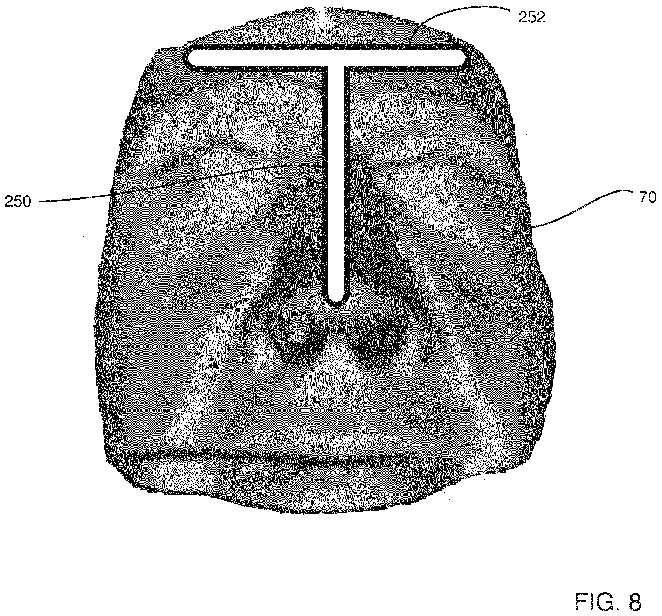

Fig. 7 is a flowchart of steps of a process implemented in system 220, and Fig. 8 illustrates one of the steps, according to an embodiment of the present invention. As for the flowchart of Fig. 2, the flowchart of Fig. 7 has an initial preparation section followed by and implementation section. In the flowchart of Fig. 7 steps 230, 232, and 234 are substantially respectively the same as steps 100, 102, and 104 of the flowchart of Fig. 2.

In an image analysis step 236, the processor analyzes surface image 70 generated in step 232, using the values acquired in steps 232 and 234, to delineate voxels within the acquired image of an upper-case "T" shape. The T shape comprises the bridge of the patient's nose as a vertical line 250 (Fig. 8) of the T. To determine voxels of the vertical line the processor starts from the nose tip determined in step 232, and finds other voxels of the bridge of the nose, i.e., the bony section of the nose, by looking for local maxima in surface image 70 in proximity to, and vertically above, the nose tip. The processor continues this process iteratively to find voxels corresponding to the complete vertical line 250 of the T shape.

To find voxels corresponding to a horizontal line 252 of the T shape, the processor selects voxels that are a preset vertical distance above a line joining the patient's eye centers, as found in step 234. In one embodiment the preset distance is 5 cm above the eye center line, but in other embodiments the distance may be larger or smaller than 5 cm. The preset distance is chosen so that the voxels of the horizontal line overlie the bony section of the patient's forehead.

If necessary, vertical line 250 of voxels, found as described above, is extended so that it meets horizontal line 252 of voxels.

It will be understood that in analysis step 236 the processor generates a subset of the set of voxels comprising surface image 70. The subset is in the general shape of an upper-case T, and the voxels of the subset 34 overlay bony sections of the patient's head. In one embodiment the bony sections correspond to voxels of the full CT image having Hounsfield unit values greater than or equal to approximately +200. Typically, both the vertical line and the horizontal line of voxels are more than one voxel wide.

Step 236 concludes the preparation section of the flowchart.

In the implementation section of the flowchart, in an implementation step 238, physician 54 moves distal end 34 of probe 28 along the bridge of the nose of patient 22, and along the forehead of the patient. In other words, the physician moves the distal end of the probe in a "T" pattern. In some embodiments a diagram such as Fig. 8 is displayed on screen 56 so as to direct the physician to move the probe in the T pattern. During the movement the physician uses controller 52 to acquire signals from sensor 32 in the probe.

In a correlation and registration step 240, the processor uses the ICP theorem to correlate the points acquired in step 238 with the subset of voxels generated in step 236. While performing the correlation the processor also registers the two frames of reference, i.e., of the magnetic system and of the CT imaging system. The theorem uses the subset of voxels as the reference set of points, and the points acquired in step 238 as the source set of points.

In a condition step 242 the processor checks if the registration performed in step 240 is sufficiently accurate, i.e., if the errors associated with the cost function generated by the ICP theorem are sufficiently small, typically below a preset threshold. If the condition returns positive, then a notice is typically provided on screen 56 informing the physician that she/he may cease acquiring points. The notice may also suggest that the physician performs a verification of the registration, such as by touching predefined positions and having the processor mark these positions on the CT image, and/or by measuring distances between such positions. If the condition returns negative, or if the verification fails, the flowchart returns to step 238 where the physician continues to acquire points with probe 28.

The inventors have found that since the reference set of points and the source set of points have the same "T" shape, and because both sets are small subsets of their respective sets, using the ICP theorem provides extremely quick and accurate registration of the magnetic system frame of reference with the CT imaging frame of reference.

### Appendix

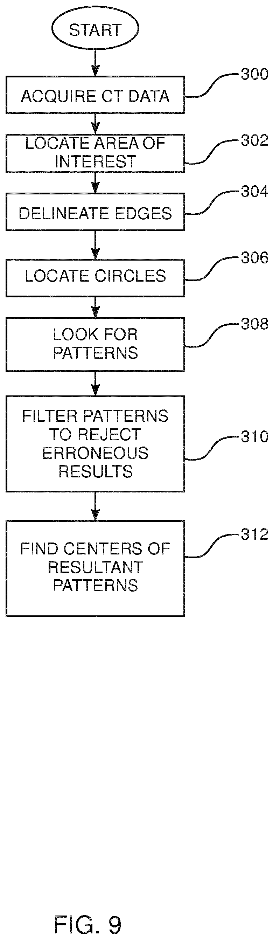

Fig. 9 is a flowchart showing steps for finding centers of the eyes of a patient in a CT image of the patient. The steps of the flowchart are based on the above-referenced article: "Locating the eyes in CT brain scan data," and in the following description the steps of the flowchart are assumed be implemented by processor 40 operating on a CT image of the head of patient 22.

An initial step 300 is substantially as step 100 described above.

In a locate area of interest step 302 processor 40 delineates a bounding box for the patient's head in the CT image, for example by raster scanning slices of the image to find the surface of the head in each slice.

In an edge detection step 304, the processor demarcates edges of entities within the bounding box, typically by using the Canny algorithm.

In a circle location step 306, the processor finds circles defined by the edges, typically by using a Hough Transform. As is known in the art, the Hough Transform uses a voting procedure to find instances of objects of a given shape, in this case circles. Typically, line voting may be used, the line being defined with respect to a circle. The results of the Hough Transform may be enhanced using the converging squares algorithm.

In a pattern recognition step 308, the circles found in step 306 are checked to see if they form a pattern, i.e., that there are corresponding circles in clusters of adjacent slices of the CT image. The ISODATA unsupervised clustering algorithm may be used to formulate clusters of circles.

In a filtration step 310, the clusters of step 308 are filtered to remove erroneous results. In one embodiment the Hough Transform may be enhanced using the converging squares algorithm.

In a pattern recognition step 308, the circles found in step 306 are checked to see if they form a pattern, i.e., that there are corresponding circles in clusters of adjacent slices of the CT image. The ISODATA unsupervised clustering algorithm may be used to formulate clusters of circles.

In a filtration step 310, the clusters of step 308 are filtered to remove erroneous results. In one embodiment the Hough Transform may be used a second time, using known possible dimensions for an eye sphere radius.

In a final step 312 the centers of the remaining clusters after step 310 are taken as the centers of the eyes in the CT image.

It will be appreciated that the embodiments described above are cited by way of example, and that the present invention is not limited to what has been particularly shown and described hereinabove. Rather, the scope of the present invention is defined by the claims.

## Claims

1. Apparatus, comprising:
a magnetic sensor (32);
a magnetic tracking system configured to measure locations of the magnetic sensor;
a probe (28) comprising the magnetic sensor that is configured to measure magnetic-system-positions of the probe in the system; and
a processor (40), configured to:
receive a computerized tomography (CT) image comprising voxels of a head of a subject (22);
generate a surface image (70) of the head of the subject;
find the highest point of the surface image that is above the coronal plane of the subject, wherein the highest point of the surface image corresponds to the nose tip of the subject;
analyze the CT image to identify respective locations of a left eye and a right eye of the subject in the CT image, so as to define a first line segment (160) joining the respective locations;
identify a voxel subset, comprising voxels, overlaying bony sections of the head, lying on:
a second line segment (252) parallel to the first line segment, by selecting voxels of the surface image that are a preset distance, in a direction away from the highest point of the surface image, from the first line segment, and
a third line segment (250) orthogonal to the first line segment, by looking for local maxima in the surface image between the highest point of the surface image and the second line segment, wherein the third line segment overlays a nose tip of the subject;
activate the magnetic tracking system;
receive magnetic-system-positions from the probe of a surface of the head overlaying the bony sections;
form a correspondence between the magnetic-system-positions from the probe and the voxel subset; and
generate a registration between the CT image and the magnetic tracking system in response to the correspondence.

2. The apparatus according to claim 1, wherein the processor (40) is configured to identify the bony sections of the head as voxels having Hounsfield unit values greater than or equal to +200.

3. The apparatus according to claim 1, wherein the second line segment (252) and the third line segment (250) form an upper-case T, the method further comprising displaying the CT image and positioning the upper-case T on the displayed CT image prior to receiving the magnetic-system-positions of the surface from the probe.

4. Apparatus, comprising:
a magnetic sensor (32);
a magnetic tracking system configured to measure locations of the magnetic sensor;
a probe (28) comprising the magnetic sensor that is configured to measure magnetic-system-positions of the probe in the system; and
a processor, configured to:
receive a computerized tomography (CT) image comprising voxels of a head of a subject (22);
generate a surface image (70) of the head of the subject;
find the highest point of the surface image that is above the coronal plane of the subject, wherein the highest point of the surface image corresponds to the nose tip of the subject;
analyze the image to identify respective locations of a left eye and a right eye of the subject in the image, so as to define a first line segment (160) joining the respective locations and a second line segment (164) orthogonally cutting the first line segment and passing through a projection of the highest point, the two line segments dividing the image into regions;
activate the magnetic tracking system;
receive magnetic-system-positions from the probe of a surface of the head;
when a count of the magnetic-system-positions of a given region exceeds a preset threshold for the given region, provide an indication thereof and form a correspondence between the magnetic-system-positions and the voxels of the image; and
generate a registration between the CT image and the magnetic tracking system in response to the correspondence.

5. The apparatus according to claim 4, wherein the processor is configured to, prior to determining the count, perform a preliminary registration between the CT image and the magnetic tracking system using magnetic-system-positions of the surface corresponding to landmark points in the CT image.

6. A method, comprising:
receiving a computerized tomography (CT) image comprising voxels of a head of a subject;
generating a surface image of the head of the subject;
finding the highest point of the surface image that is above the coronal plane of the subject, wherein the highest point of the surface image corresponds to the nose tip of the subject;
analyzing the CT image to identify respective locations of a left eye and a right eye of the subject in the CT image, so as to define a first line segment joining the respective locations;
identifying a voxel subset, comprising voxels, overlaying bony sections of the head, lying on:
a second line segment parallel to the first line segment, by selecting voxels of the surface image that are a preset distance, in a direction away from the highest point of the surface image, from the first line segment, and
a third line segment orthogonal to the first line segment, by looking for local maxima in the surface image between the highest point of the surface image and the second line segment, wherein the third line segment overlays a nose tip of the subject;
activating a magnetic tracking system configured to measure positions on the head of the subject;
positioning a probe, operative in the magnetic tracking system, in proximity to the bony sections to measure magnetic-system-positions of a surface of the head overlaying the bony sections;
forming a correspondence between the magnetic-system-positions and the voxel subset; and
generating a registration between the CT image and the magnetic tracking system in response to the correspondence.

7. The apparatus according to claim 1 or the method according to claim 6, wherein the second line segment is a preset distance above the first line segment.

8. The apparatus or the method according to claim 7, wherein the preset distance is 5 cm.

9. The method according to claim 6, and comprising identifying the bony sections of the head as voxels having Hounsfield unit values greater than or equal to +200.

10. The method according to claim 6, wherein the second line segment and the third line segment form an upper-case T, the method further comprising displaying the CT image and positioning the upper-case T on the displayed CT image prior to positioning the probe to measure the magnetic-system-positions of the surface.

11. A method, comprising:
receiving a computerized tomography (CT) image comprising voxels of a head of a subject;
generating a surface image (70) of the head of the subject;
finding the highest point of the surface image that is above the coronal plane of the subject, wherein the highest point of the surface image corresponds to the nose tip of the subject;
analyzing the image to identify respective locations of a left eye and a right eye of the subject in the image, so as to define a first line segment joining the respective locations and a second line segment orthogonally cutting the first line segment and passing through a projection of the highest point, the two line segments dividing the image into regions;
activating a magnetic tracking system configured to measure positions on the head of the subject;
positioning a probe, operative in the magnetic tracking system, in proximity to a surface of the head corresponding to the regions, so as to measure magnetic-system-positions of the surface;
when a count of the magnetic-system-positions of a given region exceeds a preset threshold for the given region, providing an indication thereof and forming a correspondence between the magnetic-system-positions and the voxels of the image;
continuing positioning the probe in proximity to a surface of the head corresponding to the regions, so as to measure magnetic-system-positions of the surface, until the preset threshold has been reached for all regions; and
generating a registration between the CT image and the magnetic tracking system in response to the correspondence.

12. The apparatus according to claim 4 or the method according to claim 11, wherein providing the indication comprises altering a visual characteristic of a sketch of the head.

13. The apparatus according to claim 4 or the method according to claim 11, wherein the regions comprise four quadrants having respective preset thresholds.

14. The method according to claim 11, and comprising, prior to determining the count, performing a preliminary registration between the CT image and the magnetic tracking system using magnetic-system-positions of the surface corresponding to landmark points in the CT image.

15. The apparatus according to claim 5 or the method according to claim 14, wherein the landmark points comprise at least two of a first point below a nose tip of the subject, a second point between the eyes of the subject, a third point on a left side of the first line segment and a fourth point on a right side of the first line segment.

## Patentansprüche

1. Vorrichtung, aufweisend:
einen Magnetsensor (32);
ein magnetisches Verfolgungssystem, das zum Messen von Positionen des Magnetsensors ausgelegt ist;
eine Sonde (28), die den Magnetsensor umfasst, der zum Messen von Magnetsystempositionen der Probe im System ausgelegt ist;
einen Prozessor (40), der ausgelegt ist zum:
Empfangen eines Computertomografie-(CT-)Bildes, das Voxel eines Kopfes eines Individuums (22) umfasst;
Erzeugen eines Oberflächenbildes (70) des Kopfes des Individuums;
Ermitteln des höchsten Punkts des Oberflächenbildes, der über der Koronalebene des Individuums ist, wobei der höchste Punkt des Oberflächenbildes der Nasenspitze des Individuums entspricht;
Analysieren des CT-Bildes, um jeweilige Positionen eines linken Auges und eines rechten Auges des Individuums im CT-Bild zu identifizieren, um ein erstes Liniensegment (160) zu definieren, das die jeweiligen Positionen verbindet;
Identifizieren eines Voxel-Teilsatzes, der Voxel umfasst, die Knochenabschnitte des Kopfs überlagern, und liegt auf:
einem zweiten Liniensegment (252) parallel zum ersten Liniensegment, durch Auswählen von Voxeln des Oberflächenbildes, die eine voreingestellte Distanz in einer Richtung vom höchsten Punkt des Oberflächenbildes entfernt sind, aus dem ersten Liniensegment, und
einem dritten Liniensegment (250) orthogonal auf das erste Liniensegment, durch Suchen nach lokalen Maxima im Oberflächenbild zwischen dem höchsten Punkt des Oberflächenbildes und dem zweiten Liniensegment, wobei das dritte Liniensegment eine Nasenspitze des Individuums überlagert;
Aktivieren des magnetischen Verfolgungssystems;
Empfangen von Magnetsystempositionen einer Oberfläche des Kopfes, welche die Knochenabschnitte überlagert, von der Sonde;
Bilden einer Entsprechung zwischen den Magnetsystempositionen von der Sonde und dem Voxel-Teilsatz; und
Erzeugen einer Deckung zwischen dem CT-Bild und dem magnetischen Verfolgungssystem in Reaktion auf die Entsprechung.

2. Vorrichtung nach Anspruch 1, wobei der Prozessor (40) zum Identifizieren der Knochenabschnitte des Kopfes als Voxel mit Werten größer oder gleich +200 in Hounsfield-Einheiten ausgelegt ist.

3. Vorrichtung nach Anspruch 1, wobei das zweite Liniensegment (252) und das dritte Liniensegment (250) einen Großbuchstaben T bilden, wobei das Verfahren ferner ein Anzeigen des CT-Bildes und Positionieren des Großbuchstabens T auf dem angezeigten CT-Bild vor dem Empfangen der Magnetsystempositionen der Oberfläche von der Sonde umfasst.

4. Vorrichtung, aufweisend:
einen Magnetsensor (32);
ein magnetisches Verfolgungssystem, das zum Messen von Positionen des Magnetsensors ausgelegt ist;
eine Sonde (28), die den Magnetsensor umfasst, der zum Messen von Magnetsystempositionen der Probe im System ausgelegt ist;
einen Prozessor, der ausgelegt ist zum:
Empfangen eines Computertomografie-(CT-)Bildes, das Voxel eines Kopfes eines Individuums (22) umfasst;
Erzeugen eines Oberflächenbildes (70) des Kopfes des Individuums;
Ermitteln des höchsten Punkts des Oberflächenbildes, der über der Koronalebene des Individuums ist, wobei der höchste Punkt des Oberflächenbildes der Nasenspitze des Individuums entspricht;
Analysieren des Bildes, um jeweilige Positionen eines linken Auges und eines rechten Auges des Individuums im Bild zu identifizieren, um ein erstes Liniensegment (160), das die jeweiligen Positionen verbindet, und ein zweites Liniensegment (164) zu definieren, welches das erste Liniensegment orthogonal schneidet und durch eine Projektion des höchsten Punkts durchtritt, wobei die beiden Liniensegmente das Bild in Regionen teilen;
Aktivieren des magnetischen Verfolgungssystems;
Empfangen von Magnetsystempositionen einer Oberfläche des Kopfes von der Sonde;
Bereitstellen, wenn eine Zahl der Magnetsystempositionen einer gegebenen Region eine voreingestellte Schwelle für die gegebene Region überschreitet, einer Anzeige davon und Bilden einer Entsprechung zwischen den Magnetsystempositionen und den Voxeln des Bildes; und
Erzeugen einer Deckung zwischen dem CT-Bild und dem magnetischen Verfolgungssystem in Reaktion auf die Entsprechung.

5. Vorrichtung nach Anspruch 4, wobei der Prozessor so ausgelegt ist, dass er eine vorläufige Deckung zwischen dem CT-Bild und dem magnetischen Verfolgungssystem unter Verwendung von Magnetsystempositionen der Oberfläche, die Orientierungspunkten im CT-Bild entsprechen, vor dem Bestimmen der Zahl durchführt.

6. Verfahren, umfassend:
Empfangen eines Computertomografie-(CT-)Bildes, das Voxel eines Kopfes eines Individuums umfasst;
Erzeugen eines Oberflächenbildes des Kopfes des Individuums;
Ermitteln des höchsten Punkts des Oberflächenbildes, der über der Koronalebene des Individuums ist, wobei der höchste Punkt des Oberflächenbildes der Nasenspitze des Individuums entspricht;
Analysieren des CT-Bildes, um jeweilige Positionen eines linken Auges und eines rechten Auges des Individuums im CT-Bild zu identifizieren, um ein erstes Liniensegment zu definieren, das die jeweiligen Positionen verbindet;
Identifizieren eines Voxel-Teilsatzes, der Voxel umfasst, die Knochenabschnitte des Kopfs überlagern, und liegt auf:
einem zweiten Liniensegment parallel zum ersten Liniensegment, durch Auswählen von Voxeln des Oberflächenbildes, die eine voreingestellte Distanz in einer Richtung vom höchsten Punkt des Oberflächenbildes entfernt sind, aus dem ersten Liniensegment, und
einem dritten Liniensegment orthogonal auf das erste Liniensegment, durch Suchen nach lokalen Maxima im Oberflächenbild zwischen dem höchsten Punkt des Oberflächenbildes und dem zweiten Liniensegment, wobei das dritte Liniensegment eine Nasenspitze des Individuums überlagert;
Aktivieren eines magnetischen Verfolgungssystems, das zum Messen von Positionen auf dem Kopf des Individuums ausgelegt ist;
Positionieren einer im magnetischen Verfolgungssystem operativen Sonde in der Nähe der Knochenabschnitte, um Magnetsystempositionen einer Oberfläche des Kopfes zu messen, welche die Knochenabschnitte überlagert;
Bilden einer Entsprechung zwischen den Magnetsystempositionen und dem Voxel-Teilsatz; und
Erzeugen einer Deckung zwischen dem CT-Bild und dem magnetischen Verfolgungssystem in Reaktion auf die Entsprechung.

7. Vorrichtung nach Anspruch 1 oder Verfahren nach Anspruch 6, wobei das zweite Liniensegment eine voreingestellte Distanz über dem ersten Liniensegment ist.

8. Vorrichtung oder Verfahren nach Anspruch 7, wobei die voreingestellte Distanz 5 cm beträgt.

9. Verfahren nach Anspruch 6 und umfassend ein Identifizieren der Knochenabschnitte des Kopfes als Voxel mit Werten größer oder gleich +200 in Hounsfield-Einheiten.

10. Verfahren nach Anspruch 6, wobei das zweite Liniensegment und das dritte Liniensegment einen Großbuchstaben T bilden, wobei das Verfahren ferner ein Anzeigen des CT-Bildes und Positionieren des Großbuchstabens T auf dem angezeigten CT-Bild vor dem Positionieren der Sonde zum Messen der Magnetsystempositionen der Oberfläche umfasst.

11. Verfahren, umfassend:
Empfangen eines Computertomografie-(CT-)Bildes, das Voxel eines Kopfes eines Individuums umfasst;
Erzeugen eines Oberflächenbildes (70) des Kopfes des Individuums;
Ermitteln des höchsten Punkts des Oberflächenbildes, der über der Koronalebene des Individuums ist, wobei der höchste Punkt des Oberflächenbildes der Nasenspitze des Individuums entspricht;
Analysieren des Bildes, um jeweilige Positionen eines linken Auges und eines rechten Auges des Individuums im Bild zu identifizieren, um ein erstes Liniensegment, das die jeweiligen Positionen verbindet, und ein zweites Liniensegment zu definieren, welches das erste Liniensegment orthogonal schneidet und durch eine Projektion des höchsten Punkts durchtritt, wobei die beiden Liniensegmente das Bild in Regionen teilen;
Aktivieren eines magnetischen Verfolgungssystems, das zum Messen von Positionen auf dem Kopf des Individuums ausgelegt ist;
Positionieren einer im magnetischen Verfolgungssystem operativen Sonde in der Nähe einer Oberfläche des Kopfes, die den Regionen entspricht, um Magnetsystempositionen der Oberfläche zu messen;
Bereitstellen, wenn eine Zahl der Magnetsystempositionen einer gegebenen Region eine voreingestellte Schwelle für die gegebene Region überschreitet, einer Anzeige davon und Bilden einer Entsprechung zwischen den Magnetsystempositionen und den Voxeln des Bildes;
Fortfahren mit dem Positionieren der Sonde in der Nähe einer Oberfläche des Kopfes, die den Regionen entspricht, um Magnetsystempositionen der Oberfläche zu messen, bis die voreingestellte Schwelle für alle Regionen erreicht wurde; und
Erzeugen einer Deckung zwischen dem CT-Bild und dem magnetischen Verfolgungssystem in Reaktion auf die Entsprechung.

12. Vorrichtung nach Anspruch 4 oder Verfahren nach Anspruch 11, wobei das Bereitstellen der Anzeige ein Ändern einer visuellen Charakteristik einer Skizze des Kopfes umfasst.

13. Vorrichtung nach Anspruch 4 oder Verfahren nach Anspruch 11, wobei die Regionen vier Quadranten mit jeweiligen voreingestellten Schwellen aufweisen.

14. Verfahren nach Anspruch 11 und umfassend ein Durchführen einer vorläufigen Deckung zwischen dem CT-Bild und dem magnetischen Verfolgungssystem unter Verwendung von Magnetsystempositionen der Oberfläche, die Orientierungspunkten im CT-Bild entsprechen, vor dem Bestimmen der Zahl.

15. Vorrichtung nach Anspruch 5 oder Verfahren nach Anspruch 14, wobei die Orientierungspunkte mindestens zwei von einem ersten Punkt unter einer Nasenspitze des Individuums, einem zweiten Punkt zwischen den Augen des Individuums, einem dritten Punkt auf einer linken Seite des ersten Liniensegments und einem vierten Punkt auf einer rechten Seite des ersten Liniensegments umfassen.

## Revendications

1. Appareil, comprenant :
un capteur magnétique (32) ;
un système de suivi magnétique configuré pour mesurer des emplacements du capteur magnétique ;
une sonde (28) comprenant le capteur magnétique qui est configuré pour mesurer des positions de système magnétique de la sonde dans le système ; et
un processeur (40), configuré pour :
recevoir une image de tomographie informatisée (CT) comprenant des voxels d'une tête d'un sujet (22) ;
générer une image de surface (70) de la tête du sujet ;
trouver le point le plus élevé de l'image de surface qui est au-dessus du plan coronal du sujet, le point le plus élevé de l'image de surface correspondant à la pointe de nez du sujet ;
analyser l'image CT pour identifier des emplacements respectifs d'un œil gauche et d'un œil droit du sujet dans l'image CT, de manière à définir un premier segment de ligne (160) joignant les emplacements respectifs ;
identifier un sous-ensemble de voxels, comprenant des voxels, recouvrant des sections osseuses de la tête, se trouvant sur :
un deuxième segment de ligne (252) parallèle au premier segment de ligne, en sélectionnant des voxels de l'image de surface qui sont à une distance prédéfinie, dans une direction s'éloignant du point le plus élevé de l'image de surface, à partir du premier segment de ligne, et
un troisième segment de ligne (250) orthogonal au premier segment de ligne, en recherchant des maxima locaux dans l'image de surface entre le point le plus élevé de l'image de surface et le deuxième segment de ligne, le troisième segment de ligne recouvrant une pointe de nez du sujet ;
activer le système de suivi magnétique ;
recevoir des positions de système magnétique en provenance de la sonde d'une surface de la tête recouvrant les sections osseuses ;
former une correspondance entre les positions de système magnétique en provenance de la sonde et le sous-ensemble de voxels ; et
générer un enregistrement entre l'image CT et le système de suivi magnétique en réponse à la correspondance.

2. Appareil selon la revendication 1, le processeur (40) étant configuré pour identifier les sections osseuses de la tête comme des voxels ayant des valeurs d'unité de Hounsfield supérieures ou égales à +200.

3. Appareil selon la revendication 1, le deuxième segment de ligne (252) et le troisième segment de ligne (250) formant un T majuscule, le procédé comprenant en outre l'affichage de l'image CT et le positionnement du T majuscule sur l'image CT affichée avant la réception des positions de système magnétique de la surface en provenance de la sonde.

4. Appareil, comprenant :
un capteur magnétique (32) ;
un système de suivi magnétique configuré pour mesurer des emplacements du capteur magnétique ;
une sonde (28) comprenant le capteur magnétique qui est configuré pour mesurer des positions de système magnétique de la sonde dans le système ; et
un processeur, configuré pour :
recevoir une image de tomographie informatisée (CT) comprenant des voxels d'une tête d'un sujet (22) ;
générer une image de surface (70) de la tête du sujet ;
trouver le point le plus élevé de l'image de surface qui est au-dessus du plan coronal du sujet, le point le plus élevé de l'image de surface correspondant à la pointe de nez du sujet ;
analyser l'image pour identifier des emplacements respectifs d'un œil gauche et d'un œil droit du sujet dans l'image, de manière à définir un premier segment de ligne (160) joignant les emplacements respectifs et un deuxième segment de ligne (164) coupant orthogonalement le premier segment de ligne et passant par une projection du point le plus élevé, les deux segments de ligne divisant l'image en régions ;
activer le système de suivi magnétique ;
recevoir des positions de système magnétique en provenance de la sonde d'une surface de la tête ;
lorsqu'un décompte des positions de système magnétique d'une région donnée dépasse un seuil prédéfini pour la région donnée, fournir une indication de celui-ci et former une correspondance entre les positions de système magnétique et les voxels de l'image ; et
générer un enregistrement entre l'image CT et le système de suivi magnétique en réponse à la correspondance.

5. Appareil selon la revendication 4, le processeur étant configuré pour, avant de déterminer le décompte, réaliser un enregistrement préliminaire entre l'image CT et le système de suivi magnétique en utilisant des positions de système magnétique de la surface correspondant à des points de repère dans l'image CT.

6. Procédé, comprenant :
la réception d'une image de tomographie informatisée (CT) comprenant des voxels d'une tête d'un sujet ;
la génération d'une image de surface de la tête du sujet ;
le fait de trouver le point le plus élevé de l'image de surface qui est au-dessus du plan coronal du sujet, le point le plus élevé de l'image de surface correspondant à la pointe de nez du sujet ;
l'analyse de l'image CT pour identifier des emplacements respectifs d'un œil gauche et d'un œil droit du sujet dans l'image CT, de manière à définir un premier segment de ligne joignant les emplacements respectifs ;
l'identification d'un sous-ensemble de voxels, comprenant des voxels, recouvrant des sections osseuses de la tête, se trouvant sur :
un deuxième segment de ligne parallèle au premier segment de ligne, en sélectionnant des voxels de l'image de surface qui sont à une distance prédéfinie, dans une direction s'éloignant du point le plus élevé de l'image de surface, à partir du premier segment de ligne, et
un troisième segment de ligne orthogonal au premier segment de ligne, en recherchant des maxima locaux dans l'image de surface entre le point le plus élevé de l'image de surface et le deuxième segment de ligne, le troisième segment de ligne recouvrant une pointe de nez du sujet ;
l'activation d'un système de suivi magnétique configuré pour mesurer des positions sur la tête du sujet ;
le positionnement d'une sonde, fonctionnant dans le système de suivi magnétique, à proximité des sections osseuses pour mesurer des positions de système magnétique d'une surface de la tête recouvrant les sections osseuses ;
la formation d'une correspondance entre les positions de système magnétique et le sous-ensemble de voxels ; et
la génération d'un enregistrement entre l'image CT et le système de suivi magnétique en réponse à la correspondance.

7. Appareil selon la revendication 1 ou procédé selon la revendication 6, le deuxième segment de ligne étant à une distance prédéfinie au-dessus du premier segment de ligne.

8. Appareil ou procédé selon la revendication 7, la distance prédéfinie étant de 5 cm.

9. Procédé selon la revendication 6, et comprenant l'identification des sections osseuses de la tête en tant que voxels ayant des valeurs d'unité de Hounsfield supérieures ou égales à +200.

10. Procédé selon la revendication 6, le deuxième segment de ligne et le troisième segment de ligne formant un T majuscule, le procédé comprenant en outre l'affichage de l'image CT et le positionnement du T majuscule sur l'image CT affichée avant de positionner la sonde pour mesurer les positions de système magnétique de la surface.

11. Procédé comprenant :
la réception d'une image de tomographie informatisée (CT) comprenant des voxels d'une tête d'un sujet ;
la génération d'une image de surface (70) de la tête du sujet ;
le fait de trouver le point le plus élevé de l'image de surface qui est au-dessus du plan coronal du sujet, le point le plus élevé de l'image de surface correspondant à la pointe de nez du sujet ;
l'analyse de l'image pour identifier des emplacements respectifs d'un œil gauche et d'un œil droit du sujet dans l'image, de manière à définir un premier segment de ligne joignant les emplacements respectifs et un deuxième segment de ligne coupant orthogonalement le premier segment de ligne et passant par une projection du point le plus élevé, les deux segments de ligne divisant l'image en régions ;
l'activation d'un système de suivi magnétique configuré pour mesurer des positions sur la tête du sujet ;
le positionnement d'une sonde, fonctionnant dans le système de suivi magnétique, à proximité d'une surface de la tête correspondant aux régions, de manière à mesurer des positions de système magnétique de la surface ;
lorsqu'un décompte des positions de système magnétique d'une région donnée dépasse un seuil prédéfini pour la région donnée, la fourniture d'une indication de celui-ci et la formation d'une correspondance entre les positions de système magnétique et les voxels de l'image ;
le fait de continuer à positionner la sonde à proximité d'une surface de la tête correspondant aux régions, de manière à mesurer des positions de système magnétique de la surface, jusqu'à ce que le seuil prédéfini ait été atteint pour toutes les régions ; et
la génération d'un enregistrement entre l'image CT et le système de suivi magnétique en réponse à la correspondance.

12. Appareil selon la revendication 4 ou procédé selon la revendication 11, la fourniture de l'indication comprenant la modification d'une caractéristique visuelle d'une esquisse de la tête.

13. Appareil selon la revendication 4 ou procédé selon la revendication 11, les régions comprenant quatre quadrants ayant des seuils prédéfinis respectifs.

14. Procédé selon la revendication 11, et comprenant, avant de déterminer le décompte, la réalisation d'un enregistrement préliminaire entre l'image CT et le système de suivi magnétique en utilisant des positions de système magnétique de la surface correspondant à des points de repère dans l'image CT.

15. Appareil selon la revendication 5 ou procédé selon la revendication 14, les points de repère comprenant au moins deux points parmi : un premier point sous une pointe de nez du sujet, un deuxième point entre les yeux du sujet, un troisième point sur un côté gauche du premier segment de ligne et un quatrième point sur un côté droit du premier segment de ligne.
